# EUROPEAN PATENT SPECIFICATION

(11) **EP 0 578 780 B1**
(45) Date of publication and mention of the grant of the patent: **02.07.2003**
(21) Application number: 92912205.9
(22) Date of filing: 02.04.1992
(51) Int. Cl.: A61K 31/616, A61P 7/02

(54) **SUPPRESSION OF THROMBOXANE LEVELS BY PERCUTANEOUS ADMINISTRATION OF ASPIRIN**
UNTERDRÜCKUNG DER THROMBOXANSPIEGEL DURCH PERKUTANE VERABREICHUNG VON ASPIRIN
ABAISSEMENT DES TAUX DE THROMBOXANE PAR ADMINISTRATION PERCUTANEE D'ASPIRINE

(30) Priority: 03.04.1991 US 680195
(43) Date of publication of application: 19.01.1994
(73) Proprietor: Keimowitz, Rudolph, M.D., Wayzata, Minnesota 55391 (US); VANDERBILT UNIVERSITY, Nashville Tennessee 37240 (US)
(72) Inventor: KEIMOWITZ, Rudolph M., Wayzata,Minnesota 55391 (US); FITZGERALD, Desmond J., Sutton, Dublin 13 (IE)
(74) Representative: Brown, John David
(86) International application number: US9202576
(87) International publication number: WO92020343

(56) References cited:
- EP-A- 0 055 635
- EP-A- 0 162 239
- WO-A-91/00096
- DE-A- 3 413 052
- FR-A- 2 295 753
- J.E.F. REYNOLDS et al.: "Martindale the Extra Pharmacopoeia", 29th edition, 1989, pages 3-8, The Pharmaceutical Press, London, GB, see the whole document: "Aspirin"
- The Journal of Clinical Investigation, vol. 69, no. 6, June 1982, P. PATRIGNANI et al.: "Selective cumulative inhibition of platelet thromboxane production by low-dose aspirin in healthy subjects", pages 1366-1372, see the whole document (cited in the application)
- CHEST, vol. 95, no. 2, February 1989, J. HIRSH et al.: "Aspirin and other platelet active drugs. Relationships among dose, effectiveness, and side effects", pages 12S-18S, see the whole document
- Yakuri to Chiryo, vol. 16, no. 1, January 1988, S.-I. NAITO et al.: "Percutaneous absorption of salicylic acid derivatives", pages 17-25, see the whole document
- The Journal of Burn Care & Rehabilitation, vol. 6, no. 6, November/December 1985, J.P. HEGGERS et al.: "Thromboxane inhibitors for the prevention of progressive dermal ischemia due to the thermal injury", pages 466-468, see the whole document

## Description

The present invention relates to the use of acetyl salicylic acid (aspirin) as an antithrombotic agent and as an agent to treat other medical conditions benefiting from suppression of thromboxane levels. Particularly, the present invention relates to the percutaneous administration of aspirin for inducing such effects and treating such conditions.

### BACKGROUND OF THE INVENTION

With the recognition of the role of antithrombotic agents in clinical medicine, investigators have pursued their efficacy, optimal dose, route of administration and safety. Aspirin has been found to be an effective antithrombotic agent in patients with cerebrovascular disease and ischemic heart disease. Aspirin may also have other antithrombotic applications. Although aspirin has become widely used as an antithrombotic agent, it still exhibits undesirable side effects, including gastrointestinal toxicity which is probably dose related.

To induce its suppressive effects, aspirin irreversibly acetylates the enzyme cyclo-oxygenase found in platelets and vascular wall cells [Burch et al., J. Clin. Invest. 61:314 (1978); Majerus, J. Clin. Invest. 72:1521 (1983); Roth et al., J. Clin. Invest. 56:624 (1975)]. Cyclo-oxygenase converts arachidonic acid to thromboxane-A₂ (TXA₂) in platelets and to prostaglandin-I₂ (PGI₂ or prostacyclin) in vascular walls [ see for example, FitzGerald et al., J. Clin. Invest. 71:676 (1983); Preston et al., N. Engl. J. Med. 304:76 (1981)]. TXA₂ induces platelet aggregation and vasoconstriction, while PGI₂ inhibits platelet aggregation and induces vasodilation. In other words, aspirin can have both an antithrombotic effect (by reducing TXA₂ production) and a thrombogenic effect (by reducing PGI₂ production). As a result, striking an appropriate balance between aspirin's effects on TXA₂ and PGI₂ production has been a goal of aspirin therapy under these circumstances.

It is generally accepted that when aspirin is administered in doses of approximately 1,000 mg/day, it inhibits both TKA₂ and PGI₂ synthesis [Weksler et al., N. Engl. J. Med. 308:800 (1983)]. Daily administration of very low doses of aspirin (approximately 40 mg/day) has been reported to inhibit thromboxane-B₂ synthesis in vitro and to reduce the urinary excretion of 2,3-dinor-thromboxane-B₂ (both of which are metabolites of TXA₂), without producing significant changes in the urinary excretion of 6-keto-prostaglandin-F₁a and 2,3-dinor-6-keto-prostaglandin-F₁a (which are both metabolites of PGI₂ production) [Patrignani et al., J. Clin. Invest. 69-1366 (1982); FitzGerald et al., supra]. While 40 mg/day has no significant effect on prostacyclin biosynthesis, it does have some measurable effect [FitzGerald et al., supra]. Moreover this dose does not suppress 2,3-dinor-TXB₂ very well and it is not known whether it suppresses bradykinin-stimulated prostacyclin formation. Therefore, this dose has not been demonstrated to provide selective inhibition of thromboxane synthesis without also inhibiting prostacyclin formation.

In contrast, others have reported that equally low doses of aspirin reduced PGI₂ synthesis by 50% in both arterial and venous tissue [Preston et al., supra], and even lower doses (20 mg/day for 1 week) have been reported to inhibit PGI₂ synthesis in both arterial and venous tissue by 50% in atherosclerotic patients [Weksler et al., supra]. It has been proposed that although this differential effect on the inhibition of TXA₂ and PGI₂ synthesis has been reported when urinary metabolites are measured to assess inhibition, there is no significant evidence for this differential effect when PGI₂ synthesis is measured by assay of vascular wall biopsy tissue or when the assays for TXA₂ and PGI₂ are performed on blood samples [Weksler et al., supra]. However, it is not possible to achieve platelet selectivity with standard oral aspirin. Inhibition of basal PGI₂ biosynthesis is similar over doses of 80-2,400 mg/day and bradykinin-stimulated PGI₂ formation is abolished on oral aspirin 75 mg/day.

Aspirin has also been found to be an effective treatment for other medical conditions which benefit from lowering of TXA₂ levels. For example, it has been reported that daily doses of aspirin given during the third trimester of pregnancy can significantly reduce the incidence of pregnancy-induced hypertension and preeclamptic toxemia in women at high risk for these disorders as a result of reductions in TXA₂ levels [Schiff et al., N. Engl. J. Med. 321:351 (1989)]. Aspirin has also been reported to provide positive effects in women at risk for pregnancy-induced hypertension. Low doses of aspirin were reported to selectively suppress maternal thromboxane levels, but only partially suppressed neonatal thromboxane, allowing hemostatic competence in the fetus and newborn [Benigni et al., N. Engl. J. Med. 321:357 (1989)]. The use of aspirin for reducing the risk of fatal colon cancer has also been proposed [Thun et al., N. Engl. J. Med. 325:1593 (1991)]. Reduction of thromboxane levels has also been suggested as a means for treating thrombosis in patients having antiphospholipid syndrome associated with lupus [Lellouche et al., Blood 78:2894 (1991)].

The use of aspirin as a thromboxane suppressant has been hampered by its tendency to cause gastric bleeding upon traditional administration of aspirin in oral dose form. Studies have reported that aspirin produces erythema of the gastric mucosa in approximately 80% of patients with rheumatic diseases, gastric erosions in approximately 40%, and gastric ulcer in 15% [Silvoso et al., Ann. Intern. Med. 91:517 (1979)]. Aspirin applied topically to gastrointestinal tissue damages gastric mucosa and induces occult gastrointestinal bleeding [Croft et al., Br. Med. J. 1:137 (1967)]. Intravenous administration of aspirin may also produce some effects on gastric mucosa which is less pronounced with parenteral than with oral administration [Grossman et al., 40:383 (1961)]. Oral administration of diluted solutions of aspirin cause considerably less bleeding than similar doses in tablet form, and aspirin solutions containing antacids with sufficient buffering capacity cause no measurable blood loss [Leonards et al., Arch. Intern. Med. 129:457 (1972)]. Enteric-coated aspirin use results in less gastric and duodenal mucosal injury than regular aspirin [Graham et al., Ann. Intern. Med. 104:390 (1986)].

It would, therefore, be desirable to provide an appropriate dosage form of aspirin which will provide thromboxane suppressing effects, preferably selective thromboxane suppressing effects, and will also avoid the adverse side effects observed with aspirin dosage forms currently employed in aspirin therapies.

Several reports have been made of the incorporation of aspirin into a various analgesic preparations. U.S. Patent No. 4,948,588 discloses the use of ether derivatives of glycerols or polyglycerols as percutaneous absorption accelerators. Analgesics, such as morphine, codeine and aspirin, are suggested as possible active agents for use with these accelerators. An example discloses incorporation of aspirin into a suppository which was administered to male rabbits.

U.S. Patent No. 4,654,209 discloses creams containing nitroglycerine and other active ingredients. Analgesics, such as aspirin, are suggested as active ingredients. An example makes a cream containing 5-15% aspirin by weight which was applied to the skin of the abdomen, thigh or back of subjects, resulting in positive blood and urine tests for the active ingredient.

U.S. Patent No. 4,476,115 discloses analgesic compositions applied to skin together with or subsequent to the application of a non-toxic water-soluble sulfite compound. Examples described the preparation of mixtures of aspirin and anhydrous sodium sulfite which was applied to the skin of a mammal and covered with a water impervious plastic sheet held in place by adhesive tape. Bioavailability was observed within 30 to 40 minutes as evidence by increased mobility of the subject and reduction of stiffness.

Although such aspirin preparations have been used for their analgesic effects, such preparations have not to applicant's knowledge been applied for therapy in which thromboxane suppression is desired.

### SUMMARY OF THE INVENTION

According to the present invention there is provided use of aspirin for the manufacture of a pharmaceutical preparation useful for suppressing thromboxane levels in a mammalian subject without affecting prostacyclin levels or resulting in gastrointestinal toxicity, the pharmaceutical preparation comprising aspirin and a support for maintaining said aspirin in a form for percutaneous absorption by said skin, wherein said aspirin is present in an amount sufficient to reduce thromboxane levels in said subject by more than 50%. Advantageously the pharmaceutical preparation is in a form for daily application to the skin for at least 4 days, especially the pharmaceutical preparation is in a form for daily application to the skin for at least 10 days.

In accordance with the present invention, thromboxane suppressing effects are provided without the gastric side effects normally associated with aspirin therapy. Aspirin is to be applied topically to a patient's skin such that it is percutaneously absorbed. The aspirin is taken into the bloodstream in quantities sufficient to inhibit TXA₂ synthesis. The present invention can be used to treat any medical condition for which suppression of thromboxane levels is beneficial, for example, to produce antithrombotic effects and to treat pregnancy-induced hypertension and preeclamptic toxemia.

The aspirin can be applied by use of a support which contains the aspirin, including without limitation suspensions, creams, solutions, patches (adhesive and non-adhesive), gels, ointments, plasters, plaques or other known forms for applying topical agents, as long as the aspirin is in a solubilized form. Articles can be made which incorporate the aspirin preparation, in some instances with a support or carrier (such as in the form of an adhesive patch).

Absorption enhancing agents and other pharmaceutical carriers can be incorporated into the aspirin preparation in accordance with known methods. Propylene glycol, with or without isopropyl or ethyl alcohol, is a preferred carrier. In addition to aspirin, other active ingredients can be incorporated into preparations for use in the present invention such as anti-arrythmics, blood pressure regulators, etc.

The aspirin content of the preparation will vary depending on the form of administration used. In one embodiment, the aspirin is to be applied at 750 mg/day at a concentration of about 9% aspirin. To achieve the desired suppression effects, aspirin is preferably to be administered over a period of several days until TXA₂ levels are reduced to minimal levels, preferably less than 50% of baseline levels, more preferably less than 10% of baseline levels, most preferably less than 5% of baseline levels. Although TXA₂ levels will be reduced almost immediately, substantial reductions in those levels are achieved and maintained by daily administration over a course of several days, preferably at least 4 days, most preferably at least 10 days.

In studying the effects of the present invention platelet cyclooxygenase activity was used as a measure of aspirin bioavailability, in addition to plasma drug levels. A preferred vehicle, propylene glycol and ethanol, is widely used as a skin permeant and was chosen to avoid ex vivo deacetylation to the inactive metabolite, salicylate. In certain preferred embodiments, aspirin applied daily induced a dose-dependent inhibition of platelet cyclooxygenase, as measured by serum TXB₂. Maximum inhibition was achieved at 10 days and exceeded 95% at the highest dose. Such a degree of suppression is preferred to sufficiently inhibit platelet function and TXA₂ biosynthesis in vivo. Inhibition of urinary TX-M followed a similar pattern. TX-M is a major enzymatic metabolite of TXB₂ and its excretion is an index of TXA₂ biosynthesis in vivo. In contrast, the vehicle alone had no effect on serum TXB₂ or urinary TX-M. Following withdrawal of therapy, serum TXB₂ and TX-M recovered gradually over a period of days. This is consistent with inhibition of platelet cyclooxygenase in vivo. As the enzyme is inhibited irreversibly, recovery of platelet TXA₂ biosynthesis parallels the formation of new platelets, a process that has a half-life of 5 days.

In contrast to the marked inhibition of TXA₂, there was little inhibition of basal or stimulated PGI₂ formation. Basal PGI-M excretion, an index of in vivo PGI₂ biosynthesis, decreased 24% by day 4 on the highest dose of dermal aspirin. No further inhibition occurred despite continued application and by day 10, PGI-M excretion remained at 83% of baseline. This may reflect the contribution of platelet endoperoxides to PGI₂ biosynthesis or local inhibition of PGI₂ biosynthesis. PGI₂ formation in response to bradykinin infusion was also unaltered. In contrast, oral aspirin 75 mg/day suppressed basal and bradykinin-stimulated PGI-M excretion, as previously demonstrated.

The preservation of vascular cyclooxygenase is consistent with the low bioavailability of the dermal aspirin. Plasma aspirin and salicylate were determined using a highly sensitive assay that can measure levels of <0.1 ng/ml. Following oral aspirin 325 mg or 162.5 mg, peak plasma aspirin levels were 2.0 and 1.3 ug/ml, respectively. In contrast, following dermal aspirin, plasma levels peaked at 237±114 ng/ml and plasma salicylate peaked at 788±114 ng/ml.

These data suggest that aspirin applied to the skin is absorbed very slowly, resulting in a delayed onset and offset of activity. Platelets passing through the site of application are inhibited by relatively high concentrations of aspirin. A similar localized platelet effect has been reported with oral aspirin, where inhibition of serum TXB₂ occurs prior to the appearance of aspirin systematically. As platelet cyclooxygenase cannot recover, cumulative inhibition of all platelets occurs over time. In contrast, little aspirin reaches the systemic circulation, so that vascular cyclooxygenase is protected. The poor systemic bioavailability of dermal aspirin presumably reflects low skin permeability and dilution and inactivation in the venous and pulmonary circulations.

Although in one subject there were no histological changes following 10 days of drug application at 250 mg/day, skin reactions were noted in 30% of the subjects, including erythema and peeling. Similar reactions occur with high concentrations of salicylate. Preliminary studies show that reactions may be avoided by alternate day application. Such regimens have been used without reactions for up to 8 weeks. Alternatively, modifications to the preparation, such as using the lactate or sodium salt of aspirin, or the vehicle, may be better tolerated. Lower concentrations and smaller doses may be feasible under occlusive conditions, which enhance drug absorption.

When the present invention is used, as demonstrated further below with respect to certain preferred embodiments, aspirin is absorbed through the skin and results in marked and selective inhibition of platelet cyclooxygenase. This approach may prove useful in patients with known peptic ulcer disease or during coincident administration of anticoagulants, such as Warfarin or heparin. The methods of the present invention should be particularly helpful in co-administration with warfarin as the high incidence of bleeding associated with oral aspirin and Warfarin is gastrointestinal and thought to be secondary to the oral aspirin effect.

Use of such aspirin preparations in accordance with the present invention provides thromboxane suppression effects, preferably selective suppression effects, without exposing the gut to high local concentrations of aspirin, which should permit its use in patients with, for example, gastric intolerance, or duodenal or gastric ulcers.

### DESCRIPTION OF THE FIGURES

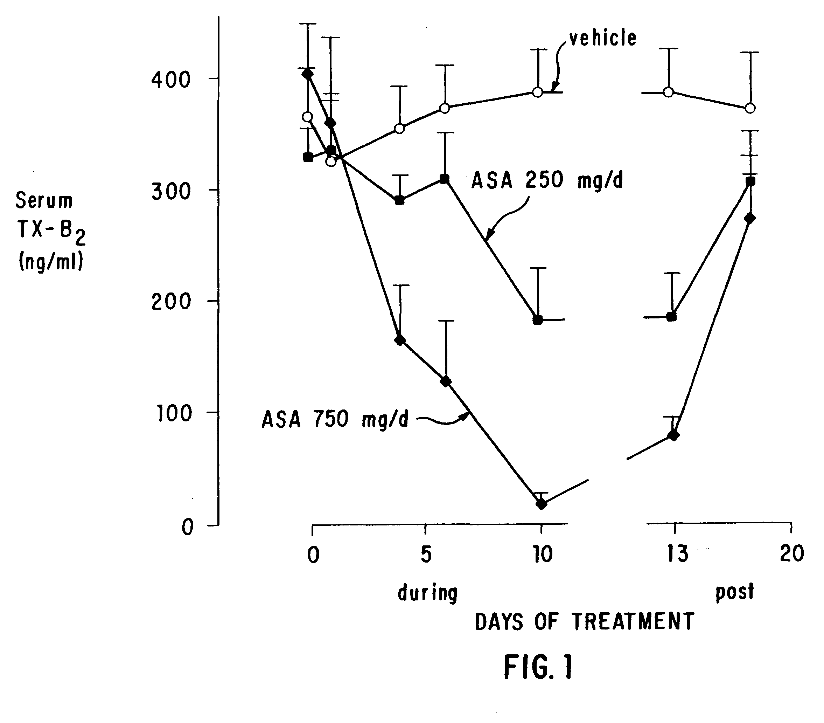

Fig. 1 is a graph of serum TXB₂ (ng/ml) levels during administration of dermal aspirin 250 mg/day and 750 mg/day vs. vehicle for 10 days and following withdrawal of therapy.

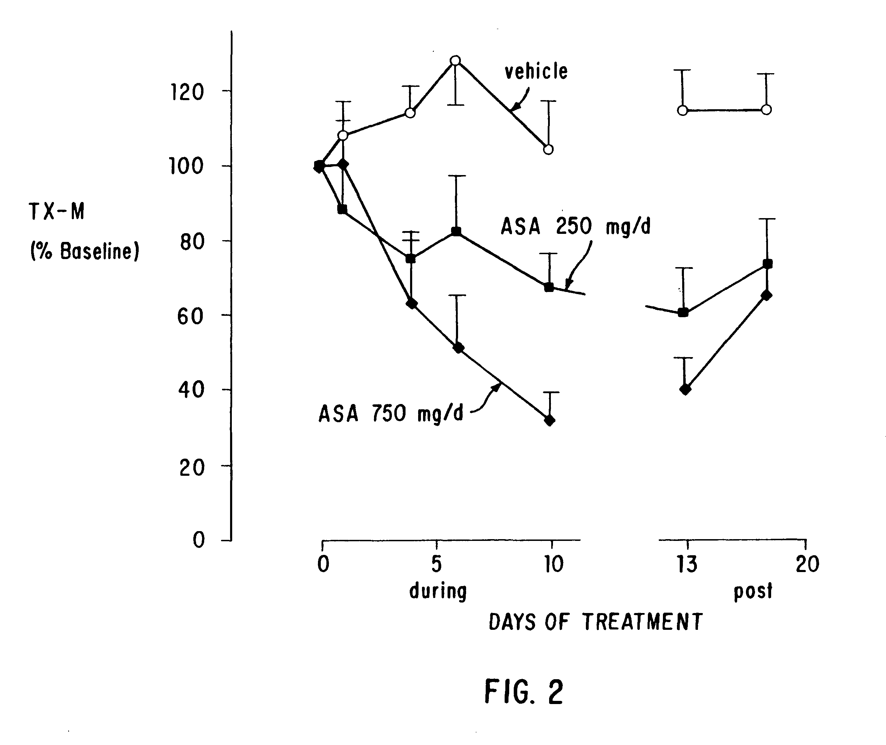

Fig. 2 is a graph of urinary excretion of 2,3-dinor TXB₂ (TX-M), expressed as a percent of baseline, during the administration of dermal aspirin or vehicle for 10 days and following withdrawal of therapy. Note that the baseline levels were 381 ± 48, 440 ± 56 and 498 ± 76 pg/mg creatinine for vehicle, aspirin 250 mg and aspirin 750 mg groups, respectively.

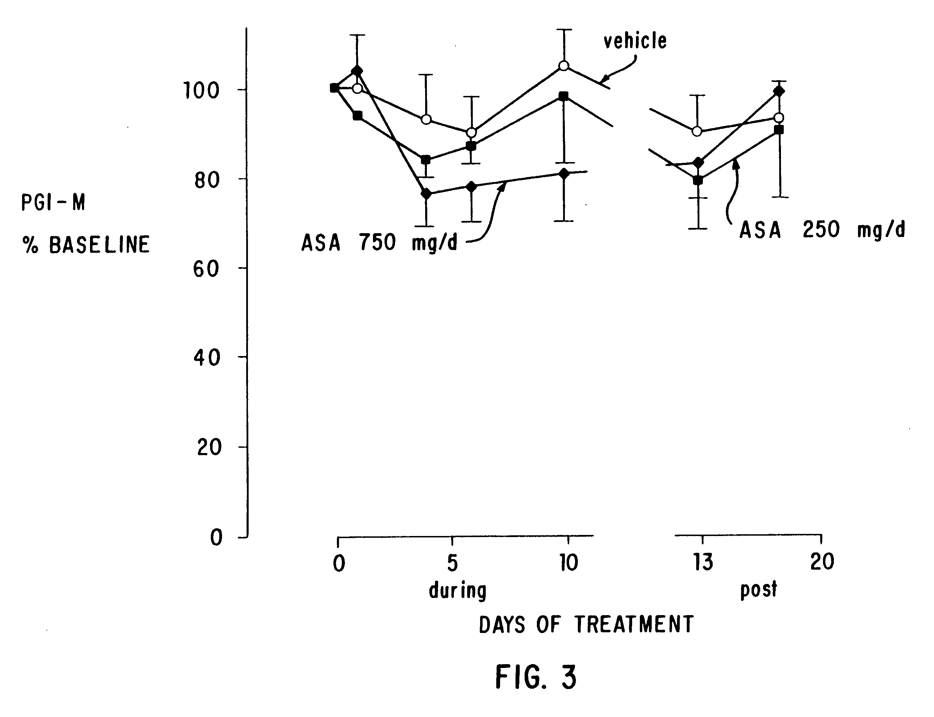

Fig. 3 is a graph of urinary excretion of 2,3-dinor-6-keto PGF₁₂ (PGI-M), expressed as a percent of baseline, during the administration of dermal aspirin or vehicle for 10 days and following withdrawal of therapy. Note that the baseline levels were 244 ± 68, 402 ± 139 and 248 ± 73 pg/mg creatinine for vehicle, aspirin 250 mg and aspirin 750 mg groups, respectively.

### DETAILED DESCRIPTION OF PREFERRED EMBODIMENTS

The advantages of the present invention can be appreciated by reference to the following example which is meant to illustrate, but not limit, the present invention.

### Example 1

Five healthy adult volunteers (3 male, 2 female) were studied. Each refrained from ingesting oral aspirin for two weeks prior to study. Prior to treatment in accordance with the present invention, baseline thromboxane levels and hemoccults were obtained.

Thromboxane levels were measured by assaying for blood levels of thromboxane-B₂ in accordance with the method described in Braden et al., Circulation 82:178 (1990). Thromboxane levels can also be measured in either blood or urine according to known methods which include those without limitation disclosed in the following references: Hirsh et al., supra; Robertson et al., N. Engl. J. Med. 304:998 (1981); Pedersen et al., N. Engl. J. Med. 311:1206 (1984); Patrignani et al., J. Clin. Invest. 69:1366 (1982); Preston et al., 304:76 (1981); Hirsh et al., N. Engl. J. Med. 304:685 (1981).

Salicylate levels were determined according to the following method. The procedure for determining salicylate is based on the formulation of a violet colored complex between ferric iron and phenols. Substances other than salicylate may react to give a positive test, but false negative results do not occur. The color reagent contains acid and mercuric ions to precipitate protein. References relevant to the assay method include: Trinder, Biochemical Journal 57:301 (1954); Tietz, Fundamentals of Clinical Chemistry, W.B. Saunders Co., 1970, pp. 882-884; Meites, Pediatric Clinical Chemistry, A.A.C.C., 1977, p. 192.

Trinder's Reagent was prepared as follows. 40 gm of mercuric chloride was dissolved in about 700 ml of deionized water by heating. The solution was cooled and 120 ml of 1N HCl and 40 gm of ferric nitrate, Fe(NO₃)₃ 9H₂O, were added. When all the ferric nitrate had dissolved, the solution was diluted to a total volume of 1000 ml with deionized water. This stock solution is stable for approximately one year.

Standards were prepared as follows. A Stock Standard (200 mg/100 ml) was prepared by dissolving 464 mg of sodium salicylate in deionized water and diluted to a total volume of 200 ml. A few drops of chloroform were added as a preservative. This standard solution is stable for approximately 6 months under refrigeration. 5, 10, 25, and 40 ml of stock standard were pipetted into a series of 100 ml volumetric flasks, diluted to a total of 100 ml with deionized water, and mixed.

0.2 ml of serum or heparinized plasma were used as sample specimens. 0.2 ml of each standard and each sample was pipetted into respectively labeled disposable polystyrene tubes. Into another polystyrene tube, 0.2 ml of deionized water was pipetted to be used as a reagent blank. 1.0 ml of deionized water was added to all tubes. 1.0 ml of Trinder's reagent was then added to all tubes, which were mixed and let stand tubes for 5 minutes. The tubes were then centrifuged for 10 minutes. The clear supernatant (minimum of 1.0 ml) wa placed into respectively labeled 10x75 nm cuvettes. Samples were analyzed by reading % T at 540 nm against the reagent blank set at 100 %T. Sample values were compared with standard values to determine levels. Results over 75 mg percent were diluted and re-analyzed.

A preparation of aspirin in isopropyl alcohol and propylene glycol was prepared by mixing "Aspirsol"™ topical aspirin (NDC 54102-001-01; commercially available from TERRI Pharmaceuticals, Inc., PO Box 6454, Kingwood, Texas 77325) in accordance with the package instructions except that 8 ml instead of 10 ml of the suspending solution was used. The resulting solution contained approximately 9% aspirin rather than the 7-8% indicated on the package label.

After base levels of thromboxane had been measured, the aspirin solution was first applied on the morning of Day 1 to the skin of the human subjects within an hour of mixing by rubbing the aspirin solution on the arms and/or chest of the subject. The application was repeated with freshly prepared solution for each of four additional mornings (Days 2-5) in the same manner. Between applications the subjects followed their normal schedule of bathing and showering. Eight hours after the fifth application (on Day 5) blood was drawn for salicylate levels and thromboxane levels to be determined. With one subject, blood was drawn for TXA₂ levels every day just before application of a new aspirin solution (i.e., approximately 24 hours after application of the previous aspirin solution). Hemoccults were also tested.

Two of the subjects continued daily application for another five days (total ten days). For Days 6 and 7, aspirin solutions freshly prepared as described above were used. Beginning with Day 8, a different aspirin preparation was used. This second preparation was prepared by crushing aspirin tablets containing approximately 975 mg aspirin to form a powder. The powder was then formed into a paste with approximately 2 ml of distilled water. This paste was then mixed with 4 ml propylene glycol and 4 ml ethanol to produce approximately 10 ml of a cloudy solution. This cloudy solution was then filtered to remove excipients and other insoluble material found in the crushed aspirin tablets. After filtering, approximately 10 ml of a clear solution was obtained which contained approximately 9% aspirin. 8 ml of the resulting solution was used in each application. This second solution was applied to the two continuing subjects as previously described. Salicylate and thromboxane levels were checked after the tenth day.

Thromboxane levels are summarized in Table 1.

**Table 1**

| Thromboxane Levels (ng/cc) | | | | | | |
|---|---|---|---|---|---|---|
| Subject | Baseline | Day 2 | Day 3 | Day 4 | Day 5 | Day 10 |
| 1 | 401 | 311 | 250 | 199 | 105/35¹ | 18 |
| 2 | 372 | | | | 105 | 12 |
| 3 | 107² | | | | 124 | |
| 4 | 402 | | | | 152 | |
| 5 | 394 | | | | 25 | |

| | | | | | | |
|---|---|---|---|---|---|---|
| ¹The first number is the level measured in the morning of Day 5 prior to administration of the new Day 5 dose. The second number is the level measured eight hours after the Day 5 application. | | | | | | |
| ²Subject 3 had a very low measured baseline thromboxane level which is believed to have been a sampling error. | | | | | | |

As summarized in the table, baseline thromboxane levels were found to range from 372-402 ng/cc in four out of five subjects. The low baseline for Subject 3 is believed to be erroneous and, as a result, the data for Subject 3 was not considered relevant. After five daily applications of aspirin in accordance with the present invention, caused a decrease in thromboxane levels of at least 50%. The two subjects that continued therapy in accordance with the present invention for another five days had marked suppression by Day 10 of 95 and 97% to levels of 18 and 12 ng/cc. Salicylate levels in four of five patients on day five were measured as 1 mg percent or less (approx. 1 mg percent being the lower limit of sensitivity of the assay,). All hemoccults taken were negative. No gastrointestinal symptoms or other side effects were noted or reported by the subjects.

### Example 2

Only healthy male and female volunteers were studied. The subjects were asked to avoid aspirin and any other cyclooxygenase inhibitors for the 10 days before and throughout the period of investigation. Aspirin (acetyl salicylic acid, USP) powder was dissolved in propylene glycol and either isopropyl alcohol or ethanol (1.7:1 v/v) to a final concentration of 94 mg/ml. Preliminary studies demonstrated that aspirin was stable in this vehicle, with less than 1% salicylate detected after 24 hr at room temperature. The aspirin preparation was made daily immediately prior to its application. Volunteers attended the clinic where the preparation was applied and were asked not to wash the area for at least 12 hours. The aspirin solution was applied to the forearm and upper arm over a 15 min interval. Volunteers received aspirin 250 mg (n=4), aspirin 750 mg (n=6) or vehicle (n=6) for 10 days and were followed for 8 days following drug withdrawal. The volunteers were aged 31-56 years, with equal numbers of male and females in each treatment group.

Blood without anticoagulant was obtained for serum TXB₂, the stable metabolite of TXA₂, prior to and at intervals during and following aspirin administration. The blood was allowed to clot in glass at 37°C for 60 min and the serum removed and stored at -20°C until analyzed. Urine was collected over 24 hours at corresponding times for measurement of 2,3-dinor-TXB₂ (TX-M) and 2,3-dinor-6-keto-PGF₁ₐ (PGI-M), major enzymatic metabolites of TXA₂ and PGI₂, respectively [Lawson et al., Analyt. Biochem. 150:463 (1985); FitzGerald et al., N. Engl. J. Med. 310: 1065 (1984)]. Excretion of these products is an index of the in vivo formation of their parent compounds [FitzGerald et al., supra; Reilly and Fitzgerald, Blood 69: 180 (1987)]. Serum TXB₂ and urinary metabolites were determined by negative ion-chemical ionization, gas chromatography-mass spectrometry (NICI-GCMS) using authentic deuterated standards, as previously described [Braden et al., supra].

Serum TXB₂, an index of the capacity of platelets to generate TXA₂, was within the normal range in all subjects prior to study, demonstrating that none had been exposed to a cyclooxygenase inhibitor. Application of the vehicle alone had no effect on serum TXB₂ in 6 subjects (Fig. 1). With aspirin 750 mg/day (n=6), there was a progressive reduction in serum TXB₂ in all but one of the volunteers. In the remaining subjects, serum TXB₂ was 5±3% of baseline by day 10 of application (n=5, p=0.003; Fig. 1). Aspirin 250 mg/day induced a smaller fall in serum TXB₂, which was 55±11% by day 10 (n=4; p < 0.01). Following the withdrawal of aspirin, serum TXB₂ increased gradually and by day 8 was 93±7 and 65±9% of baseline for aspirin 250 mg and 750 mg, respectively.

TXA₂ biosynthesis demonstrated a similar response. Thus, there was a dose dependent reduction in the urinary excretion of TX-M. At 750 mg/day of dermal aspirin. TX-M declined gradually and was 32±7% of baseline by day 10 (n=5; p=0.002) of drug application. By 8 days following drug withdrawal, excretion of the metabolite had recovered to 65±9% of the pretreatment value (Fig. 2). Despite the evidence of marked inhibition of platelet cyclooxygenase, there was only a small fall in PGI₂ biosynthesis, based on urinary PGI-M determinations (Fig. 3). Although the changes did not achieve statistical significance (p=0.074 by ANOVA), there was an apparent dose response relationship. Thus, urinary excretion of PGI-M fell to 84±4% and 76±7% of baseline on aspirin 250 mg/day and 750 mg/day, respectively (Fig. 3). The peak decrease in PGI-M excretion occurred by day 4 on both doses, in contrast to TX-M excretion.

### Example 3

In an additional 4 subjects, we examined the increase in PGI₂ formation in response to intravenous bradykinin prior to and following oral aspirin 75 mg or dermal aspirin 750 mg daily for 14 days. The protocol for bradykinin has been described previously [Clark, N.Engl.J.Med 325:1137 (1991)]. Volunteers were admitted after an overnight fast to the Clinical Research Center. Blood samples were obtained for serum TXB₂ and the subject asked to void. Through a peripheral vein, 1 liter of normal saline was infused over 1 hour. After a further hour, bradykinin was infused in incremental doses of 100-800 ng/kg/min, each over 15 min. The infusion was continued at the maximum tolerated dose for a total period of 2 hr. Blood pressure and heart rate were monitored continuously. Urine was collected in separate 2 hr aliquots prior to, during and following the bradykinin infusion.

Previous studies have demonstrated that bradykinin increases PGI₂ biosynthesis by on average 2-6 fold. In the 4 subjects studied, bradykinin induced a 5.1 ± 6 fold increase in PGI-M excretion. Two subjects were treated with oral aspirin 75 mg/day for 14 days and two with dermal aspirin 750 mg/day. Both preparations caused a marked fall in urinary TX-M (TABLE 2). Oral aspirin resulted in a decrease in urinary PGI-M at rest and following stimulation with bradykinin. In contrast, resting and stimulated PGI-M excretion was largely unaltered by dermal aspirin.

**Table 21**

| | | Dermal Aspirin (750 mg/day) | | Oral Aspirin (75 mg/day) | |
|---|---|---|---|---|---|
| | | PT 1 | PT 2 | PT 1 | PT 2 |
| TX-M | pre ASA | 220 | 121 | 136 | 111 |
| | post ASA | 46 | 29 | 26 | 43 |
| PGI-M (rest) | pre ASA | 163 | 105 | 104 | 200 |
| | post ASA | 138 | 149 | 63 | 96 |
| PGI-M (stim) | pre ASA | 1520 | 559 | 433 | 340 |
| | post ASA | 1553 | 601 | 173 | 132 |
| ¹The excretion of TX-M and PGI-M before and following dermal aspirin 750 before (rest) and following the administration of bradykinin (stim). Dermal aspirin suppressed TX-M, but had not effect on PGI-M. | | | | | |

### Example 4

In 4 subjects (2 male, 2 female) demonstrating a marked (>90%) decrease in serum TXB₂, plasma aspirin and salicylate were determined at timed intervals (0, 0.25, 0.5, 0.75, 1, 1.5, 2, 4, 6, 8, 12 and 24 hr) following the application of aspirin on days 1 and 14. Aspirin was applied in a dose of 750 mg on one limb over a 15 min interval. Samples were drawn from the opposite arm. Blood was withdrawn into heparin (10 U/ml final concentration) and potassium fluoride (5% final concentration), the latter to prevent ex vivo metabolism of aspirin by plasma esterases. The plasma was separated immediately and stored at -70°C until analyzed. Aspirin and its metabolite, salicylic acid, were measured by NICI-GCMS using deuterium-labelled analogues as internal standards, as previously described [Clark, supra].

Plasma aspirin and salicylate levels were determined following the single application of dermal aspirin in five subjects who demonstrated a marked (>90%) reduction in serum TXB₂. Plasma aspirin was barely detectable up to three hours following application when it rose to 237±114 ng/ml. At six hours, it fell to 52±14 ng/ml and by 24 hours it decreased to 4±3 ng/ml. Plasma salicylate demonstrated a similar pattern. At two hours, it was 69±20 ng/ml rising to 250±77 ng/ml at three hours. At six hours, plasma salicylate was 774±296 ng/ml. By twenty-four hours, the levels had fallen to 329±84 ng/ml. The later peak in salicylate levels is consistent with its being derived from aspirin. Moreover, this prolonged elevation of plasma salicylate is to be expected, given its longer plasma half-life. Note that levels following oral aspirin (75 mg) are 1-2 ug/ml.

## Claims

1. Use of aspirin for the manufacture of a pharmaceutical preparation useful for suppressing thromboxane levels in a mammalian subject without affecting prostacyclin levels or resulting in gastrointestinal toxicity, the pharmaceutical preparation comprising aspirin and a support for maintaining said aspirin in a form for percutaneous absorption by said skin, wherein said aspirin is present in an amount sufficient to reduce thromboxane levels in said subject by more than 50%.

2. A use according to Claim 1, wherein said carrier is selected from the group consisting of suspensions, creams, solutions, patches, gels, ointments, plasters and plaques.

3. A use according to Claim 1 or 2, wherein the pharmaceutical preparation contains about 9% aspirin.

4. A use according to Claim 1, 2 or 3, wherein the pharmaceutical preparation has a carrier comprising propylene glycol.

5. A use according to Claim 4, wherein said carrier further comprises an alcohol selected from isopropyl alcohol and ethyl alcohol.

6. A use according to any one of the preceding claims, wherein the pharmaceutical preparation further comprises at least one other active ingredient.

7. A use according to any one of the preceding claims, wherein the pharmaceutical preparation further comprises an agent for promoting absorption of said aspirin.

8. A use according to any one of the preceding claims, wherein aspirin is present in an amount sufficient to reduce serum thromboxane levels by at least 90%.

9. A use according to any one of the preceding claims, wherein aspirin is present in an amount sufficient to reduce serum thromboxane levels by at least 95%.

10. A use according to any one of the preceding claims, wherein aspirin is present in an amount which is insufficient to inhibit bradykinin-stimulated prostacyclin formation.

11. A use according to any one of the preceding claims, wherein the pharmaceutical preparation further comprises an anticoagulant.

12. A use according to any one of the preceding claims, wherein the suppression of thromboxane levels is for treating a medical condition selected from the group consisting of thrombosis, pregnancy-induced hypertension and preeclamptic toxemia.

13. A use according to any one of claims 1 to 11, wherein the suppression of thromboxane levels is for treating a subject to the risk of colon cancer in the mammalian subject.

14. A use according to any one of the preceding claims, wherein the pharmaceutical preparation is in a form for daily application to the skin for at least 4 days.

15. A use according to any one of the preceding claims, wherein the pharmaceutical preparation is in a form for daily application to the skin for at least 10 days.

## Patentansprüche

1. Verwendung von Aspirin zur Herstellung einer pharmazeutischen Zubereitung, die nützlich ist zur Unterdrückung der Thromboxanspiegel in einem Säugerindividuum, ohne daß die Prostacyclinspiegel beeinflußt werden oder sich gastrointestinale Toxizität ergibt, wobei die pharmazeutische Zubereitung Aspirin und einen Träger zum Halten besagten Aspirins in einer Form für die perkutane Absorption über besagte Haut umfaßt, wobei besagtes Aspirin in einer Menge vorliegt, die ausreichend ist, um die Thromboxanspiegel in besagtem Individuum um mehr als 50 % zu verringern.

2. Verwendung nach Anspruch 1, **dadurch gekennzeichnet, daß** besagter Träger ausgewählt ist aus der Gruppe, die aus Suspensionen, Cremes, Lösungen, Patches, Gels, Salben, Pflastern und Plaques besteht.

3. Verwendung nach Anspruch 1 oder 2, **dadurch gekennzeichnet, daß** die pharmazeutische Zubereitung etwa 9 % Aspirin enthält.

4. Verwendung nach Anspruch 1, 2 oder. 3, **dadurch gekennzeichnet, daß** die pharmazeutische Zubereitung einen Träger aufweist, der Propylenglykol umfaßt.

5. Verwendung nach Anspruch 4, **dadurch gekennzeichnet, daß** besagter Träger außerdem einen Alkohol umfaßt, der aus Isopropylalkohol und Ethylalkohol ausgewählt ist.

6. Verwendung nach einem der vorangehenden Ansprüche, **dadurch gekennzeichnet, daß** die pharmazeutische Zubereitung außerdem wenigstens einen weiteren aktiven Inhaltsstoff umfaßt.

7. Verwendung nach einem der vorangehenden Ansprüche, **dadurch gekennzeichnet, daß** die pharmazeutische Zubereitung außerdem ein Mittel zur Begünstigung der Absorption besagten Aspirins umfaßt.

8. Verwendung nach einem der vorangehenden Ansprüche, **dadurch gekennzeichnet, daß** Aspirin in einer Menge vorhanden ist, die ausreichend ist, um die Serumthromboxanspiegel um wenigstens 90 % zu verringern.

9. Verwendung nach einem der vorangehenden Ansprüche, **dadurch gekennzeichnet, daß** Aspirin in einer Menge vorhanden ist, die ausreichend ist, um die Serumthromboxanspiegel um wenigstens 95 % zu verringern.

10. Verwendung nach einem der vorangehenden Ansprüche, **dadurch gekennzeichnet, daß** Aspirin in einer Menge vorhanden ist, die ausreichend ist, um Bradykinin-stimulierte Prostacyclinbildung zu inhibieren.

11. Verwendung nach einem der vorangehenden Ansprüche, **dadurch gekennzeichnet, daß** die pharmazeutische Zubereitung außerdem ein gerinnungshemmendes Mittel umfaßt.

12. Verwendung nach einem der vorangehenden Ansprüche, **dadurch gekennzeichnet, daß** die Unterdrückung der Thromboxanspiegel zur Behandlung eines medizinischen Zustandes erfolgt, der ausgewählt ist aus der Gruppe, die aus Thrombose, schwangerschaftsinduziertem Bluthochdruck und Schwangerschaftstoxikose besteht.

13. Verwendung nach einem der Ansprüche 1 bis 11, **dadurch gekennzeichnet, daß** die Unterdrückung der Thromboxanspiegel zur Behandlung eines Individuums auf das Risiko von Colonkrebs im Säugerindividuum erfolgt.

14. Verwendung nach einem der vorangehenden Ansprüche, **dadurch gekennzeichnet, daß** die pharmazeutische Zubereitung in einer Form zur täglichen Anwendung auf die Haut über wenigstens 4 Tage vorliegt.

15. Verwendung nach einem der vorangehenden Ansprüche, **dadurch gekennzeichnet, daß** die pharmazeutische Zubereitung in einer Form zur täglichen Anwendung auf die Haut über wenigstens 10 Tage vorliegt.

## Revendications

1. Utilisation d'aspirine pour la fabrication d'une préparation pharmaceutique utile pour supprimer les taux de thromboxane chez un sujet mammifère sans affecter les taux de prostacycline ou entraîner une toxicité gastro-intestinale, la préparation pharmaceutique comprenant de l'aspirine et un support pour conserver ladite aspirine sous une forme pour une absorption percutanée par ladite peau, où ladite aspirine est présente dans une quantité suffisante pour réduire les taux de thromboxane chez ledit sujet de plus de 50 %.

2. Utilisation selon la revendication 1, dans laquelle ledit excipient est choisi à partir du groupe constitué de suspensions, crèmes, solutions, timbres, gels, pommades, plâtres et plaques.

3. Utilisation selon la revendication 1 ou 2, dans laquelle la préparation pharmaceutique contient environ 9 % d'aspirine.

4. Utilisation selon la revendication 1, 2 ou 3, dans laquelle la préparation pharmaceutique a un excipient comprenant du propylèneglycol.

5. Utilisation selon la revendication 4, dans laquelle ledit excipient comprend de plus un alcool choisi à partir d'alcool isopropylique et d'alcool éthylique.

6. Utilisation selon l'une quelconque des revendications précédentes, dans laquelle la préparation pharmaceutique comprend de plus au moins un autre ingrédient actif.

7. Utilisation selon l'une quelconque des revendications précédentes, dans laquelle la préparation pharmaceutique comprend de plus un agent pour favoriser l'absorption de ladite aspirine.

8. Utilisation selon l'une quelconque des revendications précédentes, dans laquelle l'aspirine est présente dans une quantité suffisante pour réduire les taux de thromboxane de sérum d'au moins 90 %.

9. Utilisation selon l'une quelconque des revendications précédentes, dans laquelle ladite aspirine est présente dans une quantité suffisante pour réduire les taux de thromboxane de sérum d'au moins 95 %.

10. Utilisation selon l'une quelconque des revendications précédentes, dans laquelle ladite aspirine est présente dans une quantité qui est insuffisante pour inhiber la formation de prostacycline stimulée par la bradykinine.

11. Utilisation selon l'une quelconque des revendications précédentes, dans laquelle la préparation pharmaceutique comprend de plus un anticoagulant.

12. Utilisation selon l'une quelconque des revendications précédentes, dans laquelle la suppression des taux de thromboxane est pour traiter un état médical choisi à partir du groupe constitué de thrombose, d'hypertension induite par la grossesse et d'éclampsisme.

13. Utilisation selon l'une quelconque des revendications 1 à 11, dans laquelle la suppression des taux de thromboxane est pour traiter un sujet vis-à-vis du risque de cancer du colon chez le sujet mammifère.

14. Utilisation selon l'une quelconque des revendications précédentes, dans laquelle la préparation pharmaceutique est sous une forme pour une application quotidienne sur la peau pendant au moins quatre jours.

15. Utilisation selon l'une quelconque des revendications précédentes, dans laquelle la préparation pharmaceutique est sous une forme pour une application quotidienne sur la peau pendant au moins dix jours.
